# EUROPÄISCHE PATENTANMELDUNG

(11) **EP 2 014 327 A1**
(43) Veröffentlichungstag der Anmeldung: **14.01.2009**
(21) Anmeldenummer: 08001488.9
(22) Anmeldetag: 28.01.2008
(51) Int. Cl.: A61M 16/04, A61M 25/02

(54) **Tubusfixierung**

(30) Priorität: 07.02.2007 DE 202007001749 U
(71) Anmelder: Schmitz, Susanne, 41812 Erkelenz (DE)
(72) Erfinder: Schmitz, Susanne, 41812 Erkelenz (DE)

(57) **Zusammenfassung**

Die Tubusfixierung besteht aus Fixierungsober- (1) und Fixlerungsunterteil (2), welche mit einander verbunden sein können. Das Fixierungsunterteil (2) weist innen eine Klebefläche (10) und eine Auskerbung (9) für den Blockungsmechanismus (8), außen eine Schrägverzahnung (7) und an der Unterseite eine Rinne (6) auf, die als Schiene für den Unterkiefer und gleichzeitig als Beissschutz (6) dient. Das Fixierungsoberteil (1) hat ebenfalls eine Einkerbung (6), die sich jedoch an der Oberseite befindet und als Schienung für den Oberkiefer und als Beissschutz (6) dient.
Ebenso weist auch das Fixierungsoberteil (1) eine Schrägverzahnung (7) auf. Die Schrägverzahnung (7) funktioniert beim Zusammenschieben mit dem Fixierungsunterteil (2) im Sinne einer Selbsthemmung.
Zusätzlich sind zwei Fixierungshäkchen (4) an der vorderen Oberseite des Fixierungsoberteils (1) angebracht, durch die ein zusätzliches Fixierungsband (5), welches unter dem Tubus herführt, eingeführt wird.
Es ist mit Klettverschlüssen (11) zu schließen.
Das Fixierungsband (5) teilt sich vor den Ohren in zwei Teile.
Das Fixierungsband (5) ist über den Ohren, unter den Ohren oder auch über Kreuz zu schließen.

## Beschreibung

Aufgrund der Altersentwicklung in unserer Gesellschaft sowie dem medizinischen Fortschritt nimmt die Anzahl der Menschen stetig zu, die ein hohes Alter erreichen und viele Erkrankungen aufweisen. Auf den Intensivstationen der Krankenhäuser finden sich zunehmend multimorbide Menschen, die in vielen Fällen auch künstlich beatmet werden müssen.

Zur Durchführung einer Beatmung benötigt man in der Regel einen Endotrachealtubus, der meist durch den Mund in die Luftröhre eingeführt wird und so einen sicheren Atemwegszugang darstellt. Dieser Tubus muss zwingend gut gesichert werden, da er oftmals die einzige Möglichkeit darstellt die Sauerstoffversorgung des Patienten zu gewährleisten.

Es existieren verschiedene Systeme um einen Tubus zu sichern.

Die folgende Auswahl soll einen Überblick verschaffen, hat aber nicht den Anspruch der Vollständigkeit:
a) Herkömmliche Pflaster werden verwendet, um den Tubus an der Haut des Patienten festzukleben. Das führt häufig zu Hautirritationen. Zudem verschmutzt das Pflaster sehr schnell, so dass es häufig gewechselt werden muss. Dabei birgt ein Wechsel durch die Manipulation am Tubus auch immer die Gefahr einer Verschiebung des selben. Bei stark schwitzenden Patienten oder auch stark behaarten Hautarealen ist diese Möglichkeit außerdem völlig ungeeignet, da das Pflaster hier nicht zu fixieren ist und keine Sicherung darstellt.
b) Klettfixationsbänder mit Hydrocolloldpflaster sind zwar insgesamt hautschonender, lassen sich aber ebenso wenig sicher an feuchter oder stark behaarter Haut anbringen. Verschmurzung und dadurch bedingte häufige Manipulationen sind in gleichem Maße problematisch.
c) Mullverbandswickel werden immer noch häufig am Tubus verwendet. Sie werden am Tubus mit einem Band festgebunden, das um den Kopf des Patienten geführt wird und so den Tubus inklusive Mullwickel im Mund fixiert. Zwar stellt diese Fixierung bei feuchter oder behaarter Haut kein Problem dar, der Mullwickel ist jedoch in ständigem Kontakt mit Speichel sowie Sekreten aus der Lunge oder
   auch aus dem Magen, die sich immer wieder im Mundraum befinden. Aufgrund seiner Struktur sammein sich in diesem Mullwickel viele Keime, die zu Infektionen führen können. Durch die Fixierungsbänder kommt es zudem häufig zu Einrissen der Mundwinkel, was sehr schmerzhaft für den Patienten sein kann.
d) Poisterbänder mit Klettverschlüssen sind ebenfalls bei feuchter und behaarter Haut verwendbar, jedoch sind auch sie durch den Keimbefall immer eine häufige Quelle von Infektionen.
e) Tubusflxierungen ähnlich einem Kabelbinder mit Plastikauflage am Kinn müssen ständig unterpoistert werden, um die Gefahr von Druckstellen zu vermeiden.
f) Tubusfixierungen die sich in der Hauptsache vor dem Mund befinden machen das Inspizieren und Pflegen der Mundhöhle nahezu unmöglich.

Die genannten Möglichkeiten der Fixierung eines Endotrachealtubus beinhalten verschiedene Probleme, die ihren Einsatz in der klinischen Routine limitieren und den patienten und das Pflegepersonal erheblich belasten.

Darüber hinaus fehlt bei sämtlichen genannten Methoden ein im Alltag ganz wesentlicher Aspekt: sie haben keinen Beissschutzl

Seibstverletzungen des Patienten im Bereich von Lippen und Zunge oder auch Beatmungsprobleme durch Verengung des Tubus können so nicht effektiv verhindert werden

Vor diesem Hintergrund war es das Ziel der vorliegenden Erfindung, eine Tubusfixierung bereit zu stellen, die diese bekannten Nachteile ausschließt und gleichzeitig einen effektiven Beissschutz garantiert.

Diese Aufgabe wird durch eine Tubusfixierung mit den Merkmalen des Anspruchs 1 gelöst. Vorteilhafte Ausgestaltungen sind in den Unteransprüchen enthalten.

Im folgenden wird die Erfindung mit Hilfe der Figuren 1 - 4 beispielhaft erläutert:
- Fig. 1: Schematische Darstellung einer erfindungsgemäßen Tubusfixierung von vorne links mit Integriertem Beissschutz
- Fig. 2: Seitliche Ansicht der Tubusfixierung von links
- Fig. 3: Ansicht der Tubusfixierung von oben
- Fig. 4: Ansicht Tubusfixierungs-Unterteil [2] von innen

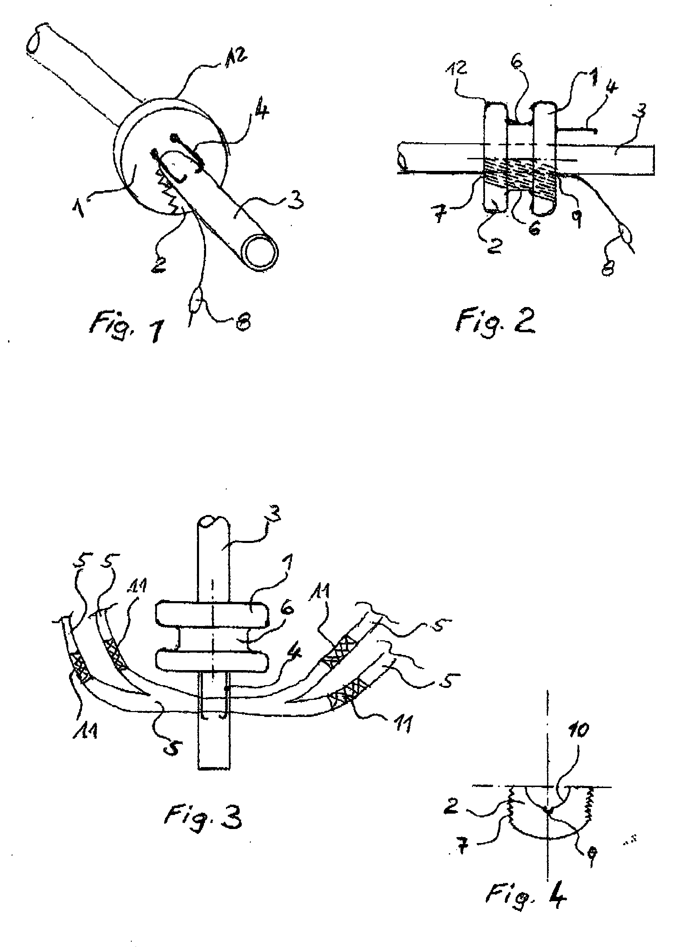

In den Figuren 1 bis 4 ist die erfindungsgemäße Tubusfixierung mit integriertem Beissschurz schematisch darstellt.

Die Tubusfixierung besteht im wesentlichen aus Fixierungs-Oberteil [1], Fixierungs-Unterteil [2],
Fixierband [5] und einem integrierten Beissschutz [6].

Da das Fixierband [5] den Tubus[3] von unten teilweise umschlingt und nur der Beissschutz [6] auf den Zähnen bzw. auf Ober- und Unterkiefer aufliegt, erhält man eine feste Arretierung zwischen den Kiefern, und somit eine Druckminderung auf Zunge und Lippe.

Fixierungs-Oberteile [1] und Fixierungs-Unterteil [2] stellen durch das Zusammenschieben eine feste Einfassung des Tubus [3] dar. Somit bleibt das Lumen des Tubus Intakt und es tritt folglich kein zusätzlicher Atemwegswiderstand auf. Beim Zusammenschieben der beiden Teile wird eine feste Position am Tubus [3] erreicht.

Durch eine Schrägstellung der Fixierungszähne [7] können verschiedene Tubusgrößen eingefasst werden. Für eine nachträgliche Lageoptimierung des Tubus [3] ist die Verzahnung [7] als Schrägverzahnung im Bereich der Selbsthemmung ausgebildet, wodurch eine lösbare Verbindung erreicht wird.

Das Fixierbad [5] garantiert die Lagesicherheit.

Im Fixierungs-Unterteil [2] befindet sich Innen eine kleine Vertiefung [9] (vgl. Fig. 4) zur Aufnahme und zum Schutz des Blockmechanismus [8] des Endotrachealtubus.

Um bereits eine Vorfixierung mit dem Tubus [3] zu erhalten, ist ein Teil der Innenseite des Fixierungs-Unterteils [2] als Klebefläche [10] angelegt.

Vorzugsweise sind Fixierungs-Oberteil [1] und das Fixierungs-Unterteil [2] miteinander verbunden.

Die abgerundeten Kanten [12] an Fixierungs-Oberteil [1] und Fixierungs-Unterteile [2] verhindern Druckulcerationen beim Patienten.

Da die gesamte Tubusfixierung mit den Fixierhaken [4] am Fixierungs-Oberteil [1] sehr klein ist, kann die Mundhöhle jederzeit vom Pflegepersonal nahezu vollständig inspiziert und gesäubert werden.
Die Mundpflege Ist somit sehr gut und einfach möglich ohne das Fixierband [5] lösen oder einen zusätzlichen Beissschutz einbringen zu müssen.

Das Fixierband [5] läst sich durch Klettverschlüsse [11] leicht öffnen und schließen. Das Fixierband [5] kann gewechselt werden ohne Gefahr das der Tubus verrutscht.

Das Fixierband [5] befindet sich unter dem Tubus [3] und schafft somit eine gleichmäßige Druckverteilung zwischen Ober- und Unterlippe. Der Abstand zu den Lippen sorgt für einen sicheren Schutz vor Verletzungen in diesem Bereich.

Das Fixierband [5] ist geteilt und läuft sowohl über- als auch unter den Ohren. Dadurch wird der Druck des Flxlerbandes [5] verteilt und lastet nicht auf einem kleinen Hautareal wo es zu Verletzungen kommen kann.

### Bezugszeichenliste

- 1: Fixierungs-Oberteil
- 2: Fixierungs-Unterteil
- 3: Tubus
- 4: Fixierhaken
- 5: Fixierband
- 6: Beisschutz
- 7: Schrägverzahnung
- 8: Block-Mechanismus
- 9: Rinne
- 10: Klebefläche
- 11: Klettverschlüsse
- 12: abgerundete Kanten

## Patentansprüche

1. Tubusfixierung bestehend aus Fixierungs-Oberteil [1] und Fixierungs-Unterteil [2], Fixierband [5] und integriertem Beißschutz [6] zur Aufnahme und zum Fixieren von Tuben, **dadurch gekennzeichnet, dass** die Tubusfixierung für verschiedene Tubengrößen einsetzbar ist und am Fixierungs-Oberteil [1] zwei Fixierhäkchen [4] für die Befestigung eines Fixierbandes [5] aufweist.

2. Tubusfixierung nach Anspruch (1), **dadurch gekennzeichnet, dass** das Fixierungs-Oberteil [1] beidseitig eine Verzahnung innen / oder außen aufweist.

3. Tubusfixierung nach Anspruch [1], **dadurch gekennzeichnet, dass** das Fixierungs-Unterteil [2] beidseitig eine Verzahnung innen / oder außen aufweist und zwar gegensätzlich zum Fixierungs-Oberteil [1].

4. Tubusfixierung nach den Ansprüchen [2] und [3] **dadurch gekennzeichnet, dass** Fixierungs-Oberteil [1] und Fixierungs-Unterteil [2] mit ihren Verzahnungen ineinander greifen.

5. Tubusfixierung nach den Ansprüchen [2] bis [4] **dadurch gekennzeichnet, dass** die Verzahnungen als Schrägverzahnungen im Bereich der Selbsthemmung ausgebildet sind.

6. Tubusfixierung nach den Ansprüchen [1] und [2] **dadurch gekennzeichnet, dass** das Fixierungs-Oberteil [1] zwei Fixierhäkchen [4] oder zwei Ösen, vorzugsweise jedoch Fixierhäkchen [4] aufweist.

7. Tubusfixierung nach den Ansprüchen [1], [2] und [6], **dadurch gekennzeichnet, dass** die Fixierhäkchen [4] aus Kunststoff oder Metall jedoch vorzugsweise aus Kunststoff bestehen.

8. Tubusfixierung nach Anspruch [1], **dadurch gekennzeichnet, dass** das Fxierungs-Unterteil [2] eine Rinne [9], zur Aufnahme eines Block-Mechanismus [8], aufweist.

9. Tubusfixierung nach Anspruch [1], **dadurch gekennzeichnet, dass** das Fixierungs-Untertell [2] eine Klebefläche [10] aufweist.

10. Tubusfixierung nach Anspruch [1], **dadurch gekennzeichnet, dass** Fixierungs-Oberteil [1] und Fixierungs-Unterteil [2] aus zwei Teilen und / oder aus einem Teil bestehen.

11. Tubusfixierung nach Anspruch [1], **dadurch gekennzeichnet, dass** Fixierungs-Oberteil [1] und Fixierungs-Unterteil [2] abgerundete Kanten [12] aufweisen.

12. Tubusfixierung nach den Ansprüchen [1] bis [10], **dadurch gekennzeichnet, dass** die Fixierung aus Kunststoff oder Metall, vorzugsweise jedoch aus Kunststoff besteht.
